(19) Europäisches Patentamt / European Patent Office / Office européen des brevets

(11) EP 1 880 675 A1

(12) EUROPEAN PATENT APPLICATION

(43) Date of publication:
23.01.2008 Bulletin 2008/04

(51) Int Cl.:
A61B 6/06 (2006.01)

(21) Application number: 07111690.9

(22) Date of filing: 04.07.2007

(84) Designated Contracting States:
AT BE BG CH CY CZ DE DK EE ES FI FR GB GR
HU IE IS IT LI LT LU LV MC MT NL PL PT RO SE
SI SK TR
Designated Extension States:
AL BA HR MK YU

(30) Priority: 13.07.2006 JP 2006192830

(71) Applicant: Konica Minolta Medical & Graphic, Inc.
Hino-shi
Tokyo 191-8511 (JP)

(72) Inventor: Shinden, Yuko
c/o Konika Minolta Medical & Graphic,Inc.,2970
Tokyo 192-8505 (JP)

(74) Representative: Gille Hrabal Struck Neidlein Prop
Roos
Patentanwälte
Brucknerstrasse 20
40593 Düsseldorf (DE)

(54) **Diagnosis information generating system, diagnosis information generating method, and diagnosis information displaying method**

(57) Image data of a phase contrast image corresponding to a high-energy zone and image data of a phase contrast image corresponding to a low-energy zone are generated by a radiation image photographing apparatus and the image data of the generated images is displayed on an image displaying apparatus.

FIG. 2

**Description**

This application is based on Japanese Patent Application No. 2006-192830 filed on July 13, 2006 in Japanese Patent Office, the entire content of which is hereby incorporated by reference.

**BACKGROUND OF THE INVENTION**

The present invention relates to a diagnosis information generating system, a diagnosis information generating method, and a diagnosis information displaying method and more particularly to a diagnosis information generating system, a diagnosis information generating method, and a diagnosis information displaying method for diagnosing deformation of a joint.

Conventionally, it is said that in the medical field, for a tissue having a low radiation absorption rate such as the soft tissue of the human body (for example, the cartilage part of a joint, synovium, breasts, etc.), it is said to be difficult to obtain an image by radiation image photographing using radiation such as X-rays. Therefore, in a diagnosis based on a radiographic image, mainly for a tissue having a high radiation absorption rate such as the bone part, radiographing is executed at a tube voltage of 40 to 150 kVp.

However, even during radiographing of such a soft tissue, on a region where the whole tissue is a soft tissue such as breasts, even if the tube voltage is set low, the radiation absorption difference will not be excessively large and an image acceptable for diagnosis can be obtained. Therefore, for radiographing of breasts, an exclusive machine such as the so-called mammography (breast radiographing apparatus) is used, and low-tube voltage radiographing at a lower tube voltage than that of ordinary radiographing, that is, low-energy radiation is used, thus radiographing by extracting a slight radiation absorption difference is executed, so that an obtained image can be used for diagnosis (refer to Patent Document 1). And, in the mammography, generally, the tube voltage is set at 22 to 38 kVp.

On the other hand, in a tissue having a low radiation absorption ratio other than breasts such as the cartilage part of a joint or synovium, the difference in the radiation absorption ratio between the cartilage part or synovium and the tissue around it is small, so that when ordinary same magnification radiographing (close contact radiographing) is executed on the radiation energy level used for radiographing hands and fingers of the human body, no contrast is formed between the cartilage part and the peripheral tissue and when they are imaged, the two cannot be discriminated. Therefore, in the radiation image photographing, it is a present condition that a lesion image can be hardly demonstrated.

The disease rate of rheumatism in Japan reaches 1% and it is now referred to as a national disease. As its initial symptom, wear of the cartilage part (cartilage destruction) is observed and when the symptom progresses, a change in the shape of the bone part is observed. Therefore, for the rheumatism, by observation of the shape of the cartilage part, the state of disease can be diagnosed and at the present stage where only an available method of treatment for this disease is stopping the progress of the state of disease, start of medical treatment by early detection is important.

However, in the present radiation image photographing, even in the radiographing of hands which is comparatively low-tube voltage radiographing, for the aforementioned reason, the tube voltage is only lowered to forty and several kVp at most for radiographing, so that radiation of energy irradiated at this tube voltage is difficult to demonstrate the soft tissue of the cartilage part and can only demonstrate the bone part (trabecula of bone). Therefore, before deformation of the bone part, no symptom can be detected and it is insufficient from the viewpoint of early detection of the rheumatism.

Therefore, to detect such a change in the soft tissue, generally, in place of the radiation image photographing, a diagnosis is made using an image obtained by MRI (magnetic resonance imaging). Further, in recent years, in the radiation image photographing, an art for taking out radiated light of the radiation traveling straight in parallel and radiographing the cartilage part using it is reported.

However, in the photographing by an MRI, from the viewpoint of expenses and time required for diagnosis, the burden imposed on a subject is severe and it is difficult to use it in the general periodic medical examination, so that a problem arises that it is difficult to periodically execute photographing and observe changes in the joints of hands and fingers with the passage of time. Further, to execute radiographing using radiated light, huge radiographing equipment is necessary, and the radiographing takes about several tens minutes, so that it is difficult to use it for diagnosis in an ordinary medical facility.

In this respect, in recent years, an art for radiographing a phase contrast image using a radiation image photographing apparatus is known (for example, refer to Patent Documents 2 and 3). By this art, an image in which the contrast of the edge portion of a radiographed subject is emphasized can be obtained.

However, in the rheumatism, changes occur in the cartilage part as an initial symptom and thereafter it grows to a symptom such as deformation of the bone part and concurrence of osteoporosis, and therefore to appropriately diagnose the rheumatism, it is necessary to demonstrate both the soft tissue of the cartilage part and the bone part. In this respect, the art disclosed in Patent Document 1 cannot execute radiographing using high-energy radiation, so that although it can demonstrate the soft tissue of the cartilage part but cannot demonstrate the internal structure of the bone part.

Further, the phase contrast radiographing arts disclosed in Patent Documents 2 and 3 make it possible to observe the soft tissue of the cartilage part but it does not make it possible to observe as far as its clear shape, and they are not sufficient to diagnose the rheumatism.

Further, to diagnose the rheumatism, it is preferable to use the arts to diagnose images of hands and fingers where the symptom appears in the earliest stage and radiographed images of hands and fingers which are particularly easily radiographed are used for diagnosis.

However, when diagnosis images of hands are taken by radiographing, images in the state where the bone part and cartilage part are overlaid are obtained, so that the condition of the cartilage part cannot be observed precisely. Therefore, at present, particularly when intending to obtain an image of the cartilage, an image is taken while the fingers of a patient (a subject) are pulled and extended. However, a problem arises that pulling the painful fingers due to the rheumatism causes the patient severe torture.

Patent Document 1: Unexamined Japanese Patent Application Publication No. 2001-91479

Patent Document 2: Unexamined Japanese Patent Application Publication No. 2002-162705

Patent Document 3: Unexamined Japanese Patent Application Publication No. 2004-248699

## SUMMARY

Therefore, the present invention was developed to solve the aforementioned problems and is intended, using the art for providing rheumatism images by the phase contrast radiation image photographing apparatus separately applied by the applicant, to provide a diagnosis information generating system, a diagnosis information generating method, and a diagnosis information displaying method for clearly distinguishing the bone part from the cartilage part and obtaining an observable image.

To solve the aforementioned problems, the diagnosis information generating system which is an embodiment of the present invention includes a radiation source for irradiating radiation to the region of a subject to be radiographed, a radiation image detector for recording a radiographic image corresponding to the radiation dose irradiated and entered from the radiation source, a radiation image photographing device for generating image data of a phase contrast image corresponding to the high-energy zone and image data of a phase contrast image corresponding to the low-energy zone, and an output device for outputting the image data generated by the radiation image photographing device.

The diagnosis information generating method which is an embodiment of the present invention includes a low-energy zone image generating step of irradiating radiation to the region of a subject to be radiographed and generating image data of a phase contrast image corresponding to the low-energy zone, a high-energy zone image generating step of irradiating radiation to the region of a subject to be radiographed and generating image data of a phase contrast image corresponding to the high-energy zone, and an image outputting step of outputting the image data generated by the low-energy zone image generating step and high-energy zone image generating step.

The diagnosis information displaying method which is an embodiment of the present invention includes a low-energy zone image generating step of irradiating radiation to the region of a subject to be radiographed and generating image data of a phase contrast image corresponding to the low-energy zone, a high-energy zone image generating step of irradiating radiation to the region of a subject to be radiographed and generating image data of a phase contrast image corresponding to the high-energy zone, and an image displaying step of displaying switchably the image data of the phase contrast image corresponding to the low-energy zone generated at the low-energy zone image generating step and the image data of the phase contrast image corresponding to the high-energy zone generated at the high-energy zone image generating step.

## BRIEF DESCRIPTION OF THE DRAWINGS

Fig. 1 is a drawing showing the constitution of the essential section of the diagnosis information generating system of this embodiment.

Fig. 2 is a side view showing the constitution of the essential section of the radiation image photographing apparatus of this embodiment.

Fig. 3 is a schematic view showing the internal constitution of the radiation image photographing apparatus of this embodiment.

Fig. 4 is a plan view when a subject puts his left hand with the back thereof upward on the hand holding part of this embodiment.

Fig. 5 is a plan view when a subject puts his left hand with the palm thereof upward on the hand holding part of this embodiment.

Fig. 6 is a plan view when a subject puts his left hand on the subject table of this embodiment.

Fig. 7 is a plan view when a subject puts his right hand on the subject table of this embodiment.

Fig. 8 is a block diagram showing the control constitution of the radiation image photographing apparatus of this embodiment.

Fig. 9 is an illustration for the principle of the phase contrast radiographing.

Fig. 10 is an illustration for the principle of the phase contrast radiographing.

Fig. 11 is a block diagram showing the control constitution of the image processing apparatus of this embodiment.

Fig. 12(a) is a drawing showing a phase contrast image corresponding to the low-energy zone and Fig. 12(b) is a drawing showing a phase contrast image corresponding to the high-energy zone.

Fig. 13 is a flow chart showing the process of this embodiment.

Fig. 14 is a table showing differences in the image visual evaluation result depending on differences in the radiographing method and differences in the energy zone in radiographing.

## DETAILED DESCRIPTION OF THE PREFERRED EMBODIMENT

Furthermore, the present invention is characterized in the following in the system and method aforementioned.

In the diagnosis information generating system aforementioned, the radiation source of the radiation image photographing apparatus is characterized in that the average radiation energy is 27.4 keV or lower when generating image data of a phase contrast image corresponding to the low-energy zone and the average radiation energy is 41.2 keV or higher when generating image data of a phase contrast image corresponding to the high-energy zone.

The diagnosis information generating method aforementioned is characterized in that the average radiation energy of the radiation irradiated is 27.4 keV or lower when generating the image data of the phase contrast image corresponding to the low-energy zone and the average radiation energy of the radiation irradiated is 41.2 keV or higher when generating the image data of the phase contrast image corresponding to the high-energy zone.

The diagnosis information displaying method aforementioned is characterized in that the average radiation energy of the radiation irradiated is 27.4 keV or lower when generating the image data of the phase contrast image corresponding to the low-energy zone and the average radiation energy of the radiation irradiated is 41.2 keV or higher when generating the image data of the phase contrast image corresponding to the high-energy zone.

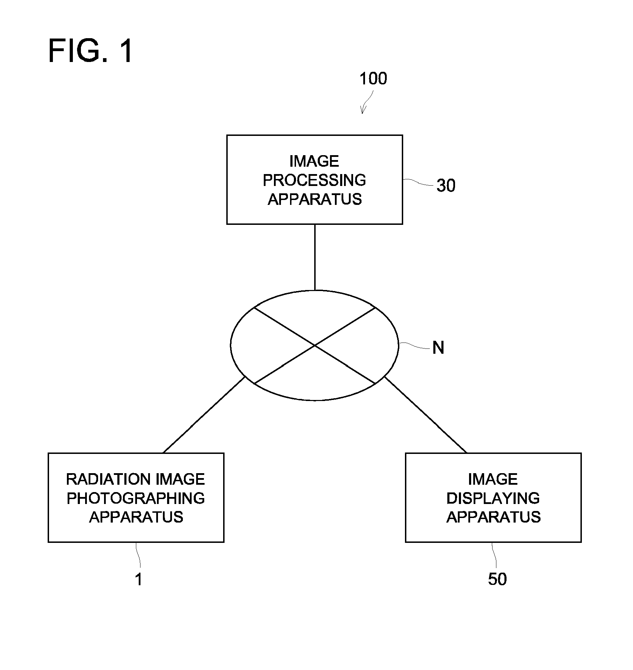

Hereinafter, by referring to Figs. 1 to 13, an embodiment of a diagnosis information generating system 100 relating to the present invention will be described. However, the scope of the present invention is not limited to the illustration.

Fig. 1 shows a constitution example of the diagnosis information generating system 100 of this embodiment. In this embodiment, the diagnosis information generating system 100 is composed of a radiation image photographing apparatus 1 as a radiographing device for generating an image to be taken by irradiating X-rays which are radiations, an image processing apparatus 30 for processing the image generated by the radiation image photographing apparatus 1, and an image displaying apparatus 50 for displaying the image processed by the image processing apparatus 30 and the respective apparatuses are connected to a communication network (hereinafter, referred to as just a network) N such as a LAN (local area network), for example, via a switching hub not drawn.

Further, the constitution of the diagnosis information generating system 100 is not limited to the one illustrated here and it may be structured, for example, so as to unite the image processing apparatus 30 and image displaying apparatus 50 and the image process is carried out and the processed image is displayed by one apparatus.

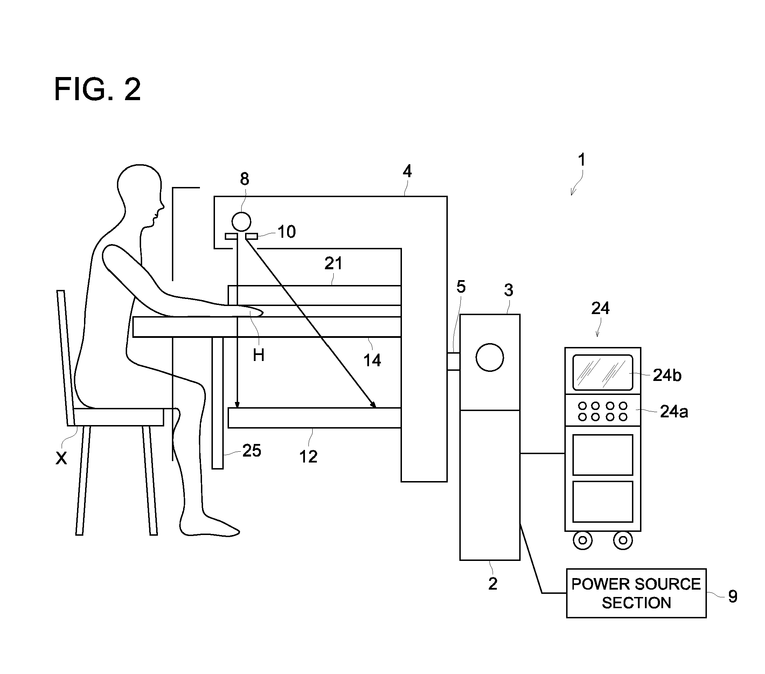

Radiation image photographing apparatus 1 will be described referring to Fig. 2 to Fig. 8.

Fig. 2 shows a structural example of radiation image photographing apparatus 1 in the present embodiment. On the radiation image photographing apparatus 1, supporting base 3 is provided on radiographing apparatus main body part 4 representing a base, in a way to be capable of rising and lowering freely relative to supporting pedestal 2. On the supporting base 3, the radiographing apparatus main body part 4 which is substantially in a shape of a rectangular parallelopiped is supported through supporting shaft 5 so that the radiographing apparatus main body part 4 may be rotated freely in the CW (clockwise) direction and in the CCW (counter clockwise) direction. The supporting base 3 is provided with drive device 6 that drives rising and lowering of the supporting base 3 and rotation of the supporting shaft 5. The drive device 6 is provided with an unillustrated known drive motor and others. The supporting base 3 and the radiographing apparatus main body part 4 are arranged to rise and lower depending on the position of subject H. The position of the subject H means a position in the vicinity of a shoulder of an examinee sitting on a chair X, and it can be adjusted to the position which allows an examinee to place an arm on subject table 14 described later and to take a posture in which the examinee hardly becomes fatigued.

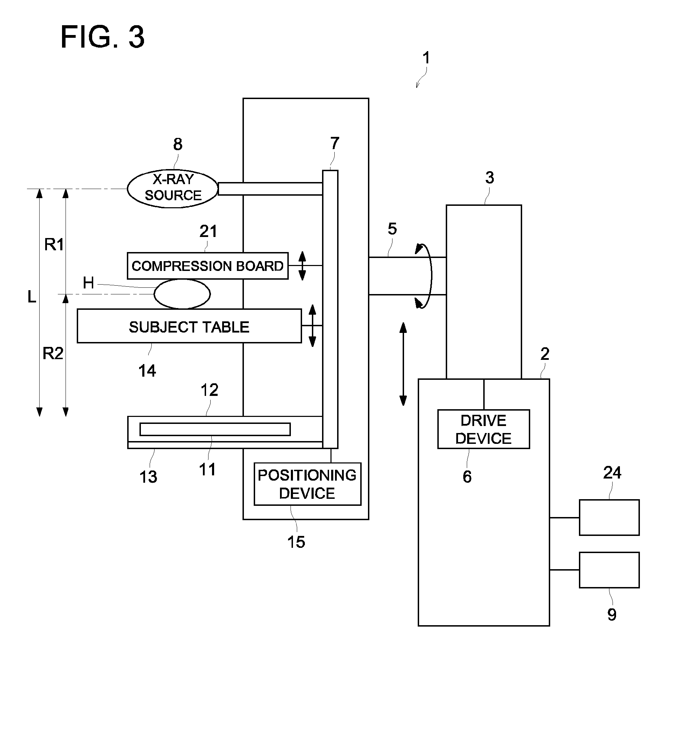

As shown in Fig. 3, holding member 7 is provided in the vertical direction in the radiographing apparatus main body part 4. On the upper part of the holding member 7, there is attached X-ray source 8 representing a radiation source that emits radiation on subject H. To the X-ray source 8, there is connected power source section 9 that applies tube voltage and tube current, through supporting shaft 5, supporting base 3 and radiographing apparatus main body part 4.

Diaphragm 10 that adjusts radiation irradiation field is provided on a radiation aperture of the X-ray source 8 on a way to open and close freely.

As the X-ray source 8, it is preferable that a rotating anode X-ray tube is used. In this rotating anode X-ray tube, X-ray is generated when an electron beam emitted from a cathode collides against an anode. This is incoherent, which is the same as natural light, and is divergent light without being parallel light X-ray. When the electron beam keeps hitting the fixed location on the anode, the anode is damaged by generated heat. Thus, in the X-ray tube used usually, the anode is rotated to prevent a decline of a life of the anode. An electron beam is caused to hit the surface in a certain area on the anode, and X-ray generated is emitted to subject H from the flat surface in the same size on the anode. The size of the flat surface viewed from the irradiating direction (subject direction) is called a focus. Focus size D ($\mu$m) means a length of one side of a square when the focus is in a form of the square, while, it means a length of a shorter side of a rectangle or of a polygon when the focus is in a form of the rectangle or the polygon and it means a diameter of a circle when the focus is in a form of the circle. When the focus size D is greater, a dose of radiation to be emitted is larger.

In this embodiment, the X-ray photographing apparatus 1 executes low-energy radiographing (image radiographing corresponding to the low-energy zone) and high-energy radiographing (image radiographing corresponding to the high-energy zone), thereby can obtain a plurality of images different in the energy zone. The X-ray photographing apparatus 1 can execute low-energy radiographing and high-energy radiographing by adjusting the set value of the tube voltage impressed to an X-ray source 8.

Further, for example, when performing the energy subtraction process for radiographing of the chest (breast image), it is general to impress a tube voltage of 60 kVp in the case of low-energy radiographing and impress a tube voltage of 120 kVp in the case of high-energy radiographing. However, even if the energy subtraction process is performed by an image obtained through such radiographing, the soft tissue of the cartilage cannot be observed.

To obtain an image on which the soft tissue can be observed, in this embodiment, the set value of the tube voltage of a power source section 9 can be adjusted so as to adjust the tube voltage to obtain an energy amount of 27.4 keV or lower and impress it to the X-ray source 8 when executing low-energy radiographing, and so as to adjust the tube voltage to obtain an energy amount of 41.2 keV or higher and impress it to the X-ray source 8 when executing high-energy radiographing.

Further, the method for obtaining a plurality of images different in the energy zone is not particularly limited and for example, a method for executing radiographing two times such as radiographing at a tube voltage of low energy and then additionally radiographing at a tube voltage of high energy, or a method for designating respectively the low-energy zone and high-energy zone, when performing the image process for image data captured by one radiographing by the image processing apparatus 30, thereby preparing two images, or a method for radiographing using two X-ray detectors 11 and simultaneously recording radiographic images carrying the high-energy component of the radiation and low-energy component in the respective X-ray detectors 11 may be used.

Further, an art relating to the radiation detector using a laminate composed of two kinds of accumulated phosphor sheets A and B which are different in the low-energy component absorption characteristic of radiation and which are laminated, wherein the sheet A having a higher low-energy component absorption characteristic is positioned at a closer position to a subject than the sheet B having a lower one is known. Permitting the radiation having transmitted through the subject to be simultaneously irradiated onto the sheets A and B, an image corresponding to the low-energy zone and an image corresponding to the high-energy zone are simultaneously obtained (for example, refer to Unexamined Japanese Patent Application Publication No. 5-211635). By use of such an art, an image corresponding to the low-energy zone and an image corresponding to the high-energy zone may be obtained.

In this embodiment, the case where both radiographing at a tube voltage of low energy and radiographing at a tube voltage of high energy are executed will be described as an example.

On the lower part of holding member 7, there is fixed one end of detector holding part 12 that holds radiation image detector 11 which detects the radiation transmitted through subject H. As the radiation image detector 11, there are given, for example, a cassette that houses a stimulable phosphor sheet, a screen (intensifying screen)/film and FPD (flat panel detector). In the present embodiment, the radiation image detector 11 whose size is 14 x 17 (inches) is used. Further, a relative position between X-ray source 8 and detector holding part 12 is fixed, and its distance is assumed to be represented by L.

A method can be employed so that both of the distance from X-ray source 8 to the subject and the distance from the subject to the radiation image detector 11 are variable according to a radiographing magnification without fixing L to be constant.

Radiation dose detecting section 13 that detects a dose of emitted radiation is provided on the bottom surface of the detector holding part 12 at the lower part of the holding member 7.

Between the X-ray source 8 and the detector holding part 12, there is provided flat-plate-shaped subject table 14 that holds examinee's fingers representing subject H from underneath, with its one end being fitted on the holding member 7. The subject table 14 is connected with positioning device 15 that is equipped with a motor for changing a position relative to the holding member 7, for adjustment of shooting magnification (adjustment of a position in the direction of a height) in the case of phase contrast radiographing.

The subject table 14 is formed to be protruded toward the examinee side beyond the other edge of the detector holding part 12. Overt the subject table 14, there is provided compression board 21 that presses and fixes subject H from above, with its one end being fitted on the holding member 7. The compression board 21 can move freely along the holding member 7, while keeping its posture to be in parallel with the subject table 14. Any of automatic movement and manual movement can be applied to the movement of the compression board 21. An edge of the compression board 21 is arranged to be protruded slightly toward the examinee side beyond the X-ray source 8 and the radiation image detector 11 (edge of effective image) which are arranged substantially vertically. Therefore, if a range of a target object to be radiographed (for example, a right hand) of an examinee is arranged to be closer to the holding member 7 than the compression board 21 is, image missing for an area of interest (a range to be radiographed) is not caused, which is preferable. It is further preferable that an edge of subject table 14 is made to be in a form of curved surface and an aged examinee having an average figure can lean its upper half of the body over the subject table 14 when the examinee sits on chair X.

In the present embodiment, on the bottom surface of subject table 14, protector 25 is installed extending substantially vertical so that the subject can take a radiographing location without hitting the leg against the detector holding part 12. Owing to this, the examinee can be positioned at a radiographing location without hitting the detector holding part 12 with his or her leg under the condition of sitting on chair X. The compression board or protector 25 are not essential component parts and the constitution is possible without using the compression board or protector 25.

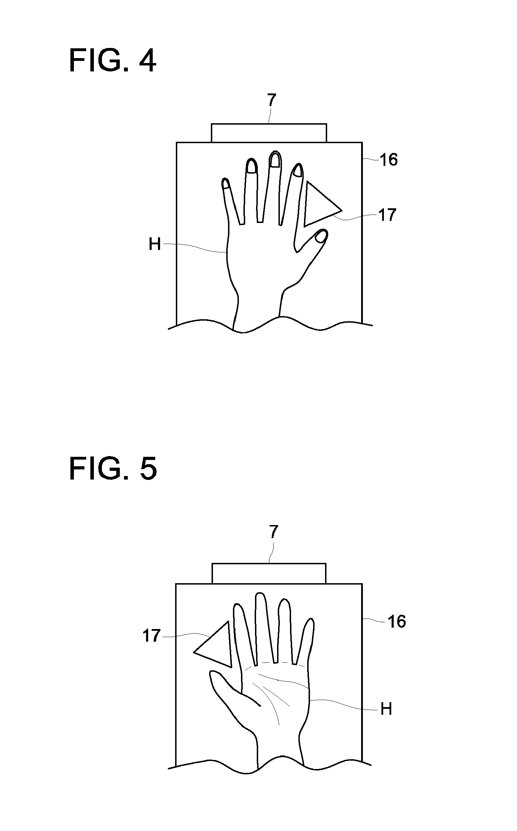

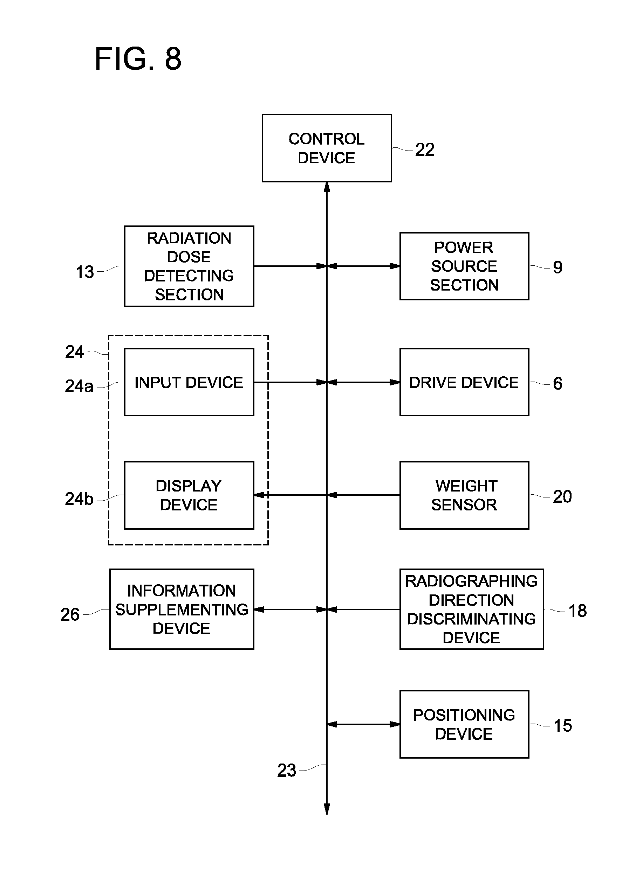

As shown in Fig. 4 and Fig. 5, hand-holding member 16 that holds the fingers of an examinee is provided on the subject table 14 to intersect with a radiation irradiation path. A size of the hand-holding member 16 is not limited in particular, provided that fingers of an examinee can be placed. On the upper surface of the hand-holding member 16, there is provided triangle magnet 17 that is arranged between the thumb and a forefinger when an examinee places fingers on the hand-holding member 16. On the hand-holding member 16, there is provided radiographing direction discriminating device 18 (see Fig. 8) that detects a location where triangle magnet 17 is placed and discriminates a position of the thumb of the examinee as the radiographing direction information.

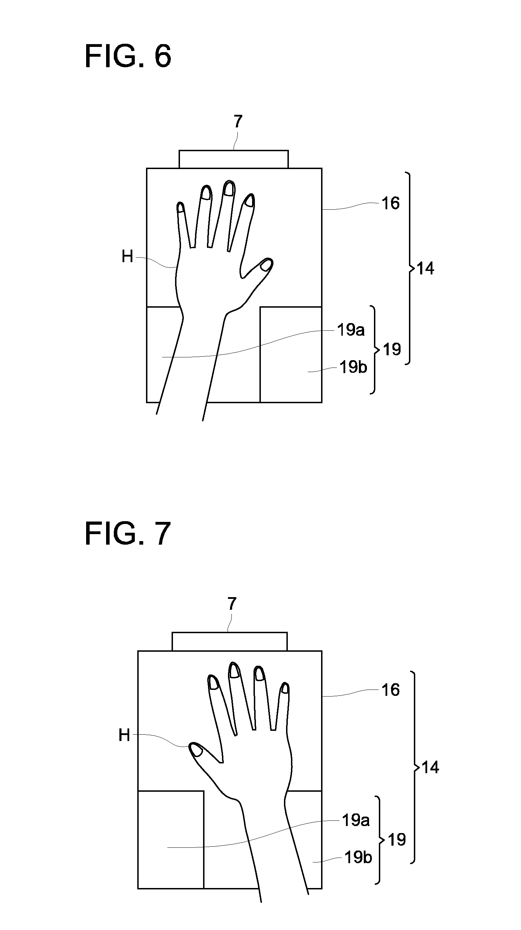

As shown in Fig. 6 and Fig. 7, arm-holding member 19 that holds an arm of an examinee is provided on the position that is closer to the examinee side than the hand-holding member 16 of the subject table 14 is. On the arm-holding member 19, there are provided left-arm-holding member 19a and right-arm-holding member 19b, and the examinee can place either a left arm or a right arm depending on photographing conditions. A size of the arm-holding member 19 is not limited in particular, and the examinee can fix its fingers stably and sufficiently if the arm below an elbow of the examinee can be placed. On each of the left-arm-holding member 19a and the right-arm-holding member 19b, there is provided weight sensor 20 (see Fig. 8) as a discriminating device for right and left that determines a right hand or a left hand of the examinee (information about right and left) depending on which one of the left-arm-holding member 19a and the right-arm-holding member 19b the arm portion of the examinee is placed on. With respect to the weight sensor 20, those which are widely known can be used without any restriction, and the number of weight sensors 20 and installation positions for them are not restricted in particular.

Radiographing direction information acquired by the radiographing direction discriminating device 18 and information for right and left acquired by weight sensor 20 are outputted to information supplementing device 26 through control device 22 which will be described later. The information supplementing device 26 may also correlate radiographing direction information and/or information for right and left to image data of phase contrast images to be generated as supplementary information. Supplementary information is not limited to those mentioned above, and ID information of an examinee may also be supplemented.

As shown in Fig. 8, radiographing apparatus main body part 4 is equipped with control device 22 that is composed of CPU (Central Processing Unit), ROM (Read Only Memory) and RAM (Random Access Memory). Radiation dose detecting section 13, power source section 9, drive device 6, positioning device 15, weight sensor 20, information supplementing device 26 and radiographing direction discriminating device 18 are connected to the control device 22 through bus 23. Further, operation device 24 having input apparatus 24a equipped with a key board and touch panel (not shown) which conduct input of radiographing conditions and a position adjusting switch for adjusting a position of subject table 14 and display device 24b such as CRT display and a liquid crystal display are connected to the control device 22. The radiographing apparatus main body part 4 can be also equipped with an information acquiring device for acquiring patient information by reading barcode or the like.

In a ROM of a control device 22, the control program for controlling each unit of the radiation image photographing apparatus 1 and various processing programs are stored, and a CPU functions as an image data generating section for controlling overall the operation of each unit of the radiation image photographing apparatus 1 in cooperation with the control program and various processing programs, executing phase contrast radiographing, and generating image data of a phase contrast image.

For example, CPU controls drive device 6 based on results of the discrimination by weight sensor 20 and radiographing direction discriminating device 18 and on radiographing conditions for an examinee, then, causes radiographing apparatus main body part 4 to rise and lower to the height adjusted to the examinee's height, and rotates supporting shaft 5 for adjusting an irradiation angle of radiation. Then, it adjusts a position of subject table 14 by positioning device 15, and adjusts a magnification rate of phase contrast radiographing. After that, the radiographing apparatus main body part 4 conducts radiographing processing, by causing power source section 9 to apply tube voltage and tube current on X-ray source 8 to irradiate subject H with radiation, and when a dose of radiation inputted from the radiation dose detecting section 13 arrives at the dose of radiation set in advance, it causes power source section 9 to stop irradiation of radiation from X-ray source 8.

To an information supplementing device 26, as mentioned above, radiographing direction information obtained by a radiographing direction discriminating device 18 and information for right and left obtained by a weight sensor 20 are outputted via the control device 22. Further, in this embodiment, from an operating device 24 or an information obtaining device not drawn, patient information (subject information) concerning a subject H, information on a radiographing time (radiographing time information), and region information concerning the radiographing region, that shows which region of the patient is the radiographed subject H are inputted, and the inputted information is outputted to the information supplementing device 26 via the control device 22. Further, when the control device 22 has a timer function, even if the radiographing time information is not inputted newly, it may be designed to permit the control device 22 to automatically obtain the radiographing time when radiographing the subject, and output the radiographing time to the information supplementing device 26 as radiographing time information to be supplemented to the concerned image data. Further, when a plurality of images different in the energy zone are obtained, for example, common identification information is given to the data of the plurality of images, thus even if the plurality of images are taken several times, it is indicated that they are to be subject to the image subtraction process.

The information supplementing device 26 makes these various information (radiographing direction information, information for right and left, subject information, radiographing time information, region information, etc.) correspond to the image data of the generated phase contrast image as supplementary information. Further, the supplementary information to be supplemented to the image data by the information supplementing device 26 is not limited to it. For example, ID information of a patient (a subject) may be supplemented. Further, the function of the information supplementing device 26 is not limited to supplementing of all the information illustrated here but it may supplement any of these information.

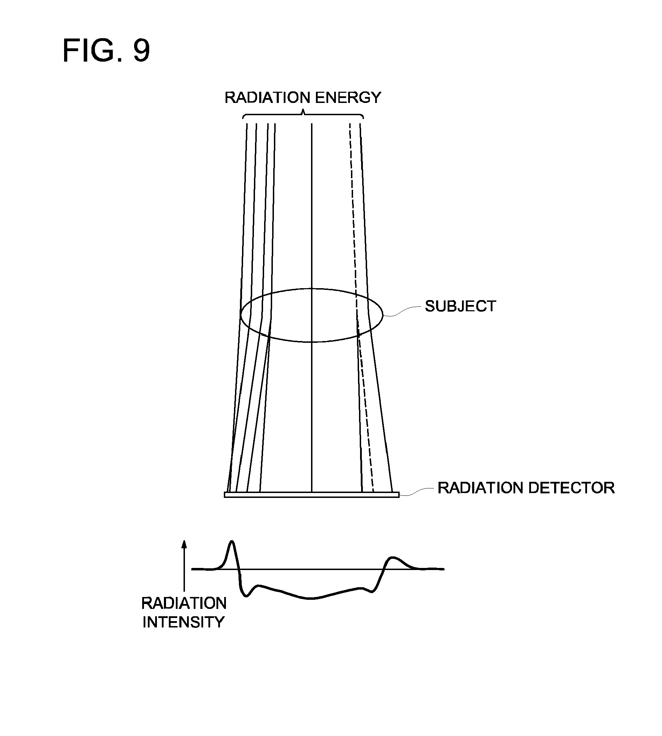

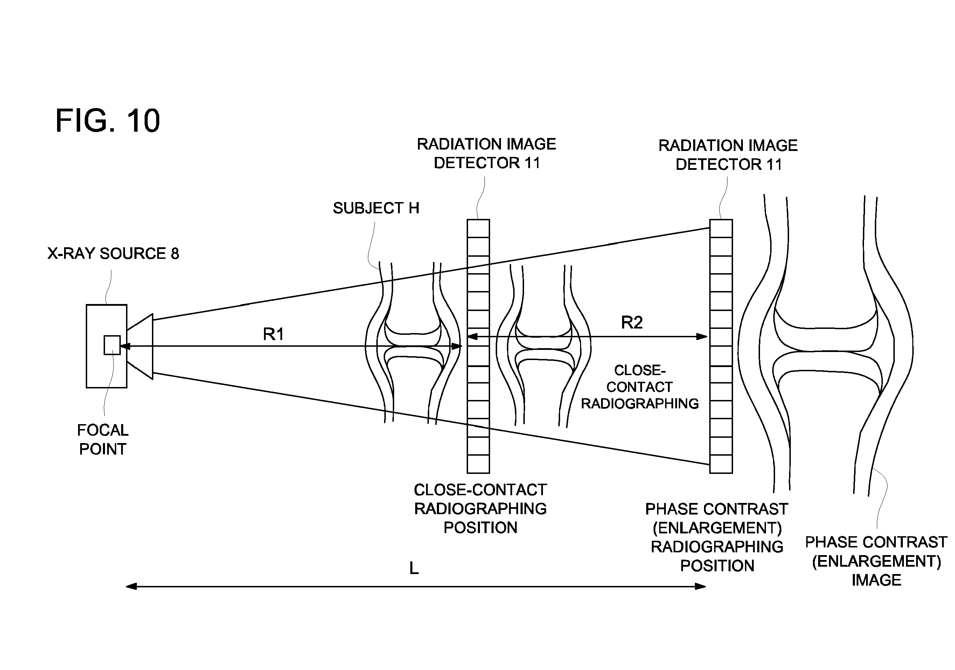

Now, a principle of phase contrast radiographing will be explained as follows, referring to Figs. 9 and 10. The phase contrast radiographing is a radiographing in which an edge-emphasized (refraction-contrast-enhanced) image caused by refraction of radiation is obtained as shown in Fig. 9 by providing certain distance R2 between subject H and radiation image detector 11. As is drawn schematically in Fig. 9, when a radiation is transmitted through an object, the radiation density is lowered by refraction on the inside of boundary of the object, while, the radiation density is enhanced further on the outside of the object because of overlapping of the radiation with other radiation which is not transmitted through the object. Thus, an edge representing a boundary portion with a subject is emphasized as an image. This is a phenomenon caused by a difference of the refractive index for radiation between an object and air. The image obtained through these phenomena is an edge-emphasized image.

Furthermore, the edge emphasis includes not only the edge emphasis on the boundary between air and a subject shown in Fig. 9 as a principle but also similar edge emphasis is caused on the boundary portion between the parts different in the refractive index even in a material. The subject boundary portion relating to the present invention can express the boundary portion between substances different in the refractive index of radiation. The components such as the bone part, cartilage part, and joint fluid existing at the joint portion of the human body are all different in the refractive index of radiation, so that by emphasizing the edge through phase contrast radiographing, an image having a clear boundary of each component (subject boundary portion) can be obtained.

Further, as shown in Fig. 10, in the ordinary radiographing method, the subject H is arranged at the position where the radiation image detector 11 makes contact with the subject H (the close contact radiographing position shown in Fig. 10). In this case, an X-ray image (a latent image) recorded in the radiation image detector 11 is of a size almost equal to the life size (the same size as that of the subject H).

On the other hand, the phase contrast radiographing provides a distance between the subject H and the radiation image detector 11 and by X-rays irradiated from the X-ray source 8 in the cone beam shape, a latent image of an enlarged X-ray image (hereinafter, referred to as an enlarged image) from the life size is detected by the radiation image detector 11.

An enlargement rate M of the enlarged image to the life size in the phase contrast radiographing, assuming the distance from the X-ray source 8 to the subject H as R1 and the distance from the subject H to the radiation image detector as R2, can be obtained by Formula (1) indicated below.

$$M = (R1 + R2)/R1 \dots\dots\dots\dots\dots\dots\dots\dots\dots\dots (1)$$

With respect to R1, the starting point thereof is the position of the focal point of the X-ray source 8 and on an ordinary X-ray source 8 on sale, the place is displayed clearly. Further, the ending point thereof is the central line of the subject H fixed by a subject table 14 for fixing the subject position. In this case, the position at the equal distance from the subject table 14 and a compression board 21 is assumed as the central line of the subject H. With respect to R2, the starting point is the central line of the subject H and the ending point is the uppermost flat surface for receiving radiation of the radiation image detector, that is, the uppermost surface of a detector holding section 12. The value of R1 + R2 coincides with a distance L between the X-ray source 8 and the detector holding section 12.

When the distance R2 between the subject H and the radiation image detector is long (when the distance R1 from the X-ray source 8 to the subject H is short), the image enlargement rate is increased and the edge emphasis effect is increased.

Further, the edge emphasis effect varies depending on the tube voltage impressed to the X-ray source 8 and as the tube voltage impressed to the X-ray source 8 increases, the edge emphasis effect when the phase contrast radiographing is executed is reduced. On the other hand, as the tube voltage impressed to the X-ray source 8 decreases, the edge emphasis effect when the phase contrast radiographing is executed is increased.

In this embodiment, a phase contrast image corresponding to the high-energy zone where a tube voltage impressed to the X-ray source 8 is 41.2 keV or higher as average radiation energy and a phase contrast image corresponding to the low-energy zone where a tube voltage impressed to the X-ray source 8 is 27.4 keV or lower as average radiation energy are taken.

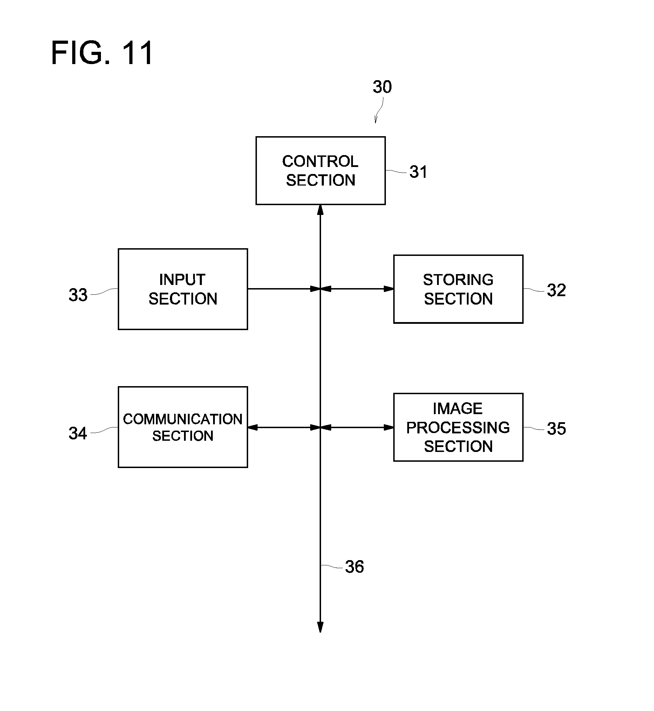

Next, by referring to Fig. 11, the image processing apparatus 30 of this embodiment will be described.

The image processing apparatus 30 relating to the present invention performs the image process to the data of the radiographic image generated by the radiation image photographing apparatus 1 and generates an image suited to diagnosis. The image processing apparatus 30, as shown in Fig. 11, is composed of a control section 31, a storing section 32, an input section 33, a communication section 34, and an image processing section 35 and these units are connected to each other via a bus 36.

The control section 31 includes a CPU (central processing unit), a RAM (random access memory), and a ROM (read only memory) (these are not drawn). The CPU uses a predetermined area of the RAM as an operation area, according to various programs stored in the ROM or the storing section 32, sends a control signal to the aforementioned units, thereby intensively controls the entire operation of the image processing apparatus 30, and executes various processes such as the image extraction process which will be described later.

The storing section 32 includes fixedly or removably storing media not drawn such as magnetic or optical storing media such as an HDD (a hard disc drive) and an optical disk and a semiconductor memory, and stores an image processing program, various programs relating to the image processing apparatus 30, and various data used when executing these processing programs.

Further, in this embodiment, in the storing section 32, data of a radiographic image which has been taken by the radiation image photographing apparatus 1 and been sent to the image processing apparatus 30 is stored. In this embodiment, the image data of the radiographic image, as described above, is sent to the image processing apparatus 30 in the state where the radiographing direction information, information for right and left, subject information, radiographing time information, and region information are supplemented as supplementary information by the information supplementing device 26 of the radiation image photographing apparatus 1, and the storing section 32 is a storing device for storing these pieces of information in the state where they are supplemented to the image data.

The input section 33 is composed of, for example, a keyboard including a cursor key, numeral input keys, and various function keys and a pointing device such as a mouse, none of which are drawn, and image processing conditions can be inputted. The input section 33 outputs an instruction signal inputted by the key operation on the keyboard and mouse operation to the control section 31.

The communication section 34 is composed of a network interface or the like and transmits and receives data between itself and an external apparatus such as the radiation image photographing apparatus 1 or the image displaying apparatus 50 connected to a network N via a switching hub. Namely, the communication section 34, via the network N, receives the image data of the radiographic image generated by the radiation image photographing apparatus 1 and properly transmits image data of an image for which the image processing is completed to an external apparatus such as the image displaying apparatus 50.

The image processing section 35 performs the image processes such as the gradation process of adjusting the contrast of an image for image data of a radiographic image, the process of adjusting the density, and the frequency process of adjusting the sharpness. By executing these, the image processing suited to the conditions according to the radiographing region can be performed.

Further, it is preferable to store beforehand the image processing parameters for specifying the image processing conditions corresponding to the conditions such as the radiographing region, radiographing condition, and radiographing direction in the storing section 32. When performing the image process, it is preferable to read the image processing parameter corresponding to it from the storing section 32 by the image processing section 35, and to decide the image processing conditions on the basis of the read parameter, according to the information supplemented to the image data such as the radiographed region and radiographing direction showing which region of the body is radiographed for the radiographic image. Further, when the information such as the radiographed region and radiographing direction is not supplemented to the image data, it is possible to input necessary condition from the input section 33 and perform the image processing on the basis of it.

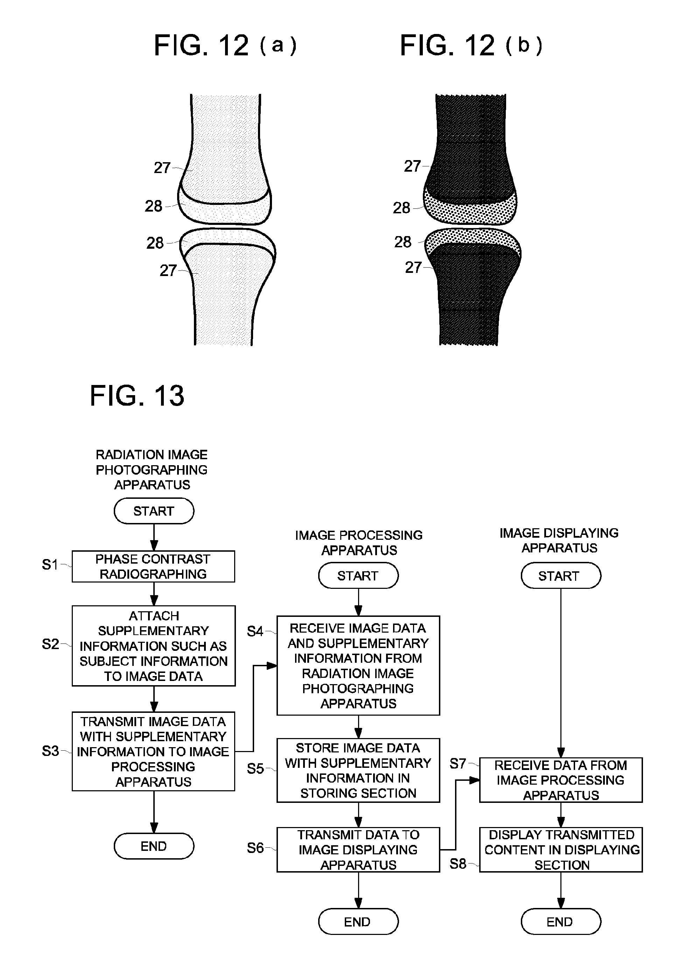

Figs. 12(a) and 12(b) express schematically an image obtained by the image processing by the image processing section 35 for the data of the radiographic image of a bone part 27 and a cartilage part 28 radiographed as a subject H.

Fig. 12(a) shows a phase contrast image corresponding to the low-energy zone among images different in the energy zone, and the outline of the cartilage part 28 including the boundary between the bone part 27 and the cartilage part 28 is clear by the edge emphasis effect. Fig. 12(b) shows a phase contrast image corresponding to the high-energy zone and clear image information of the entire bone part 27 is obtained.

Further, the image processing section 35 may be able to perform the image reduction process for forming an image at the same magnification as the subject H for a phase contrast image (enlarged image) radiographed by the radiation image photographing apparatus 1.

The reduction factor when reducing the phase contrast image radiographed with high magnification is a reduction magnification for reducing the radiographed image to the same magnification as the subject H, that is, an inverse number of the enlargement rate of the radiographed image. Namely, for example, assuming the enlargement rate of the radiographed image as A, when reducing it to the life size (the same magnification as that of the subject H), the image is reduced to 1/A times. Further, when the enlargement rate information at time of radiographing is supplemented as supplementary information of the image data, the reduction factor is calculated on the basis of this supplementary information. Further, when the enlargement information at time of radiographing is not included in the supplementary information, the image is reduced at a preset reduction factor as default or reduced at a reduction factor set by a user.

The phase contrast image is obtained by enlargement radiographing, so that the subject H on the image is large. From the viewpoint of diagnosis, an image of a familiar size of the same magnification as the subject H may be more convenient. Therefore, it is preferable for the image processing section 35 to be able to perform the image reduction process for reducing the phase contrast image as necessary. Particularly, it is desirable to output the image of life size of the subject H. Further, even if the image reduction factor is set by default as mentioned above, needless to say, it can be changed by the desire of a user.

Next, the image displaying apparatus 50 is composed of, for example, a monitor (display section) such as a CRT (cathode ray tube) or an LCD (liquid crystal display), a communication section for connecting with an external device, and a power section for supplying power, which are not drawn. The communication section is composed of a network interface and transmits and receives data between it and an external device such as the radiation image photographing apparatus 1 and image displaying apparatus 50 which are connected to the network N via the switching hub.

The image displaying apparatus 50, when the communication section 34 receives the image data of the radiographic image processed by the image processing apparatus 30 via the network N, displays properly the image on the display section. For example, when the data of the phase contrast image corresponding to the low-energy zone (for example, Fig. 12(a)) and the phase contrast image corresponding to the high-energy zone (for example, Fig. 12(b)) are transmitted from the image processing apparatus 30, the image displaying apparatus 50 displays the image on the display section. Further, the image displaying apparatus 50, when receiving singular or plural image data from the image processing apparatus 30, permits to display the plural images one by one or in parallel. The image data may be displayed by superimposing information supplemented as supplementary information.

Further, the image displaying apparatus 50 displays a medical image for diagnosis and submits it for diagnosis of a doctor, so that it is preferable to install a higher resolution monitor (display section) than a general personal computer (PC).

Next, by referring to Fig. 13, the diagnosis information generating method and diagnosis information displaying method realized by the diagnosis information generating system 1 of this embodiment will be described.

Firstly, when a subject (patient) executes inspection registration (radiographing order registration) at an inspection reception not drawn and the radiographing order information is registered, the inspection subject loads either of the left and right arms on the subject table 14 on the basis of the radiographing order information and loads a triangular magnet 17 between the thumb and the forefinger along them.

After placing of subject H is completed, the subject's arm portion placed on arm-holding member 19 is discriminated by weight sensor 20 whether it is a left hand or a right hand. In detailed explanation, the results of discrimination of the presence or absence of load of weight sensor 20 are outputted to control device 22. The control device 22 determines so that any of the left-arm-holding member 19a and right-arm-holding member 19b having more detections of load detected by weight sensor 20 provided on the left-arm-holding member 19a and right-arm-holding member 19b, is one on which the arm portion of the subject is placed. The control device 22 outputs the results of the discrimination to radiographing direction discriminating device 18.

After determining whether the subject's hand placed is a left hand or a right hand, the radiographing direction discriminating device 18 determines which of the back of the hand and the palm of the hand of the subject placed on hand-holding member faces upward, and determines an orientation of the hand representing the radiographing direction. In detailed description, the radiographing direction discriminating device 18 determines a position of the thumb based on the position of setting of triangle magnet 17 and determines an orientation of the hand of the subject by matching it with the results of discrimination by weight sensor 20. The results of discrimination are outputted to the control device 22, and an appropriate radiation irradiation angle is calculated. Incidentally, when there are available image data of examinee's fingers taken through radiographing in the past, information for right and left and/or information of radiographing direction as supplementary information is extracted, and when it does not agree with the setting this time, that situation may be given as a warning on display device 24b. Further, when there are not available image data of examinee's fingers taken through radiographing in the past, supplementary information may be established on control device 22 as one of radiographing order information for the examinee.

Thereafter, by a drive device 6 and a positioning device 15, adjustment of the position of the subject table 14 and adjustment of the angle of a radiographing apparatus main body part 4 are executed in accordance with the radiographing conditions such as the radiation irradiation angle and irradiation distance. In this embodiment, the position of the subject table 14 (refer to Figs. 3 and 10) is adjusted for phase contrast radiographing. After adjustment of the position and angle of the subject table 14, the power source section 9 impresses a tube voltage and a tube current of high energy of average radiation energy of 41.2 keV or higher to the X-ray source 8, and the X-ray source 8 irradiates radiation toward the subject H, thus radiographing is executed. Then, the power source section 9 impresses a tube voltage and a tube current of low energy of average radiation energy of 27.4 keV or lower to the X-ray source 8, and the X-ray source 8 irradiates radiation toward the subject H, thus radiographing is executed. As a result, data of two phase contrast images different in the energy zone are generated (Step S1).

When the image data of the phase contrast image are generated, to each of the generated image data, the radiographing direction information, information for right and left, subject information, radiographing time information, and region information are supplemented as supplementary information (Step S2). And, the radiation image photographing apparatus 1 transmits the generated image data to the image processing apparatus 30 together with the supplementary information (Step S3).

The image processing apparatus 30, when receiving the image data and supplementary information thereof from the radiation image photographing apparatus 1 (Step S4), preserves (stores) the received image data and supplementary information thereof in the storing section 32 (Step S5).

And, the control section 31 transmits the image data and supplementary information thereof which are transmitted from the radiation image photographing apparatus 1 to the image displaying apparatus 50 via the communication section 34 (Sep S6).

The image displaying apparatus 50, when receiving the data from the image processing apparatus 30 (Step S7), permits the display section to display the received contents (Step S8). Further, in this embodiment, the image displaying apparatus 50, when receiving, from the image processing apparatus 30, the image data of the phase contrast image corresponding to the low-energy zone (for example, Fig. 12(a)) and the image data of the phase contrast image corresponding to the high-energy zone (for example, Fig. 12(b)), can permit the display section to display these images by switching them properly. Namely, for example, when observation of the situation of the soft tissue of the cartilage part is intended by a user such as a doctor (for example, intending to judge existence of an occurrence of the initial symptom of the rheumatism), the image displaying apparatus 50 permits the display section to display the data of the phase contrast image corresponding to the low-energy zone, and for the situation of the bone part (for example, intending to judge the existence of an occurrence of osteoporosis or the existence of deformation of the bone part), permits the display section to display the data of the phase contrast image corresponding to the high-energy zone.

Further, the image data is transmitted to the image displaying apparatus 50 as a phase contrast image (enlarged image), and the image displaying apparatus 50 may permit the display section to display each image as an enlarged image, or may permit the display section to display the reduced image after the image data reduced to the same size (life size) by the image processing apparatus 30 is transmitted.

From the aforementioned, according to the diagnosis information generating system 100 of this embodiment and the diagnosis information generating method and diagnosis information displaying method which are realized by use of the system, two kinds of data of the phase contrast image producing the edge emphasis effect corresponding to the high-energy zone and low-energy zone are generated, so that by use of the radiation image photographing apparatus 1 used for the general group medical examination (periodic medical checkup), existence of deformation of the soft tissue of the cartilage part can be judged. Therefore, there is no need to use a specific apparatus such as an MRI and the effects can be produced such that an excessive burden such as expenses is not imposed on a subject and the initial symptom of the rheumatism can be diagnosed easily and precisely.

Furthermore, the image displaying apparatus 50 can permit the display section to switch properly and display the obtained image data of the phase contrast image corresponding to the low-energy zone and the obtained image data of the phase contrast image corresponding to the high-energy zone, so that a user such as a doctor, when intending to observe the situation of the soft tissue of the cartilage part, permits the display section to display the data of the phase contrast image corresponding to the low-energy zone and when intending to observe the situation of the bone part, permits the display section to display the data of the phase contrast image corresponding to the high-energy zone, thereby can diagnose easily both soft tissue and bone part.

Further, in this embodiment, the case that the image processing apparatus 30 and image displaying apparatus 50 are installed individually is described as an example. However, a constitution may be used so that the image processing device, storing device, and display device as an output device are installed in one apparatus and the image processing apparatus 30 and image displaying apparatus 50 are served as one apparatus.

Further, in this embodiment, a constitution is used so that the image displaying apparatus 50 that is a display device for displaying an image is installed in the diagnosis information generating system 100, as an output device. However, the output device is not limited to the display device. For example, in addition to the image displaying apparatus 50 or in place of the image displaying apparatus 50, it is possible to connect a printer for printing image data on a medium such as a film or paper to the network N as an output device and to print and output image data which is taken by the radiation image photographing apparatus 1 and is processed by the image processing apparatus 30.

**EXAMPLES**

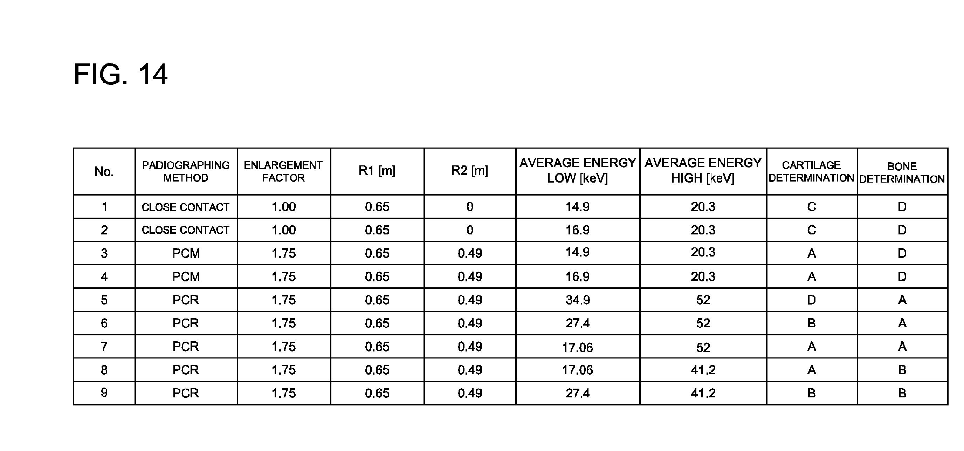

Next, by referring to Fig. 14, the diagnosis information generating system, diagnosis information generating method, and diagnosis information displaying method relating to the present invention will be described concretely by referring to the examples. Further, the present invention is not limited to these examples.

In this embodiment, as a radiation image photographing device in the example for executing a part of the present invention and the comparative examples, a phase contrast radiographing prototype for rheumatism image radiographing using a W tube as an X-ray source, and capable of executing the phase contrast radiographing as a radiographing method is used and as a comparative example, a commercial breast radiographing apparatus (mammography) using an Mo tube (molybdenum tube) as an X-ray source, and capable of executing the close-contact radiographing and phase contrast radiographing of 1.75 times as a radiographing method is used.

Further, in the columns of the radiographing method shown in Fig. 14, "Close contact" means close-contact radiographing, and "PCM" means phase contrast radiographing using an Mo tube (molybdenum tube), and "PCR" means phase contrast radiographing using a W tube (tungsten tube). Further, the Mo tube (molybdenum tube) cannot execute X-ray irradiation at high energy.

As a subject equivalent to the region to be radiographed in the present invention, a prototype finger simulated phantom (in a water solution of hyaluronate sodium filled in an acrylic case, a cartilage phantom and a bone phantom including a wound pattern are contained) is used.

As an image processing apparatus (image reading apparatus) having the image processing device of the present invention, Regius model 190 manufactured by Konicaminolta, Inc. is used and as an output apparatus equivalent to the output device of the present invention, DRYPRO model 793 is used.

Further, the tube voltage of the X-ray source at the time of radiographing, in the case of an Mo tube, the tube voltage [kVp] (average energy [keV]) is 22 kVp (14.9 keV), 28 kVp (16.9 keV), and 38 kVp (20.3 keV). Further, in the case of a W tube, the tube voltage [kVp] (average energy [keV]) is 20 kVp (17.06 keV), 40 kVp (27.4 keV), 60 kVp (34.9 keV), 80 kVp (41.2 keV), and 120 kVp (52 keV). Further, the focus diameters at the time of radiographing were all 100 $\mu$m.

The enlargement factor for radiographing is 1 times (same magnitude) and 1.75 times and the distances R1 and R2 at each enlargement factor (refer to Figs. 3 and 10), in the case of 1 times (same magnitude), are respectively R1 = 0.65 m and R2 = 0 m and in the case of 1.75 times, they are respectively R1 = 0.65 m and R2 = 0.49 m.

Fig. 14 is a table for indicating differences in the visual evaluation results of the images between the cartilage phantom and the bone phantom depending on the difference of radiographing method and the difference in the radiographing energy zone of radiographing, when a subject is radiographed under the experimental conditions aforementioned.

In Fig. 14, "Average energy high" means the average radiation energy (average X-ray energy) on the high energy side for the image subtraction process and "Average energy low" means the average radiation energy (average X-ray energy) on the low energy side for the image subtraction process. Further, "Cartilage determination" means determination for the shape and pattern of the cartilage phantom equivalent to the cartilage part and "Bone determination" means determination for the shape and pattern of the bone phantom equivalent to the bone part.

In this embodiment, the determination for visual evaluation means results of observation of the finger simulated phantom in the images by seven image estimators and the evaluation standards are assumed as "A" when the peripheral shape and pattern shape can be recognized clearly, "B" when the shape and pattern can be recognized, "C" when existence of the pattern in the bone part and the cartilage phantom in the cartilage part can be seen so as to be found, and "D" when the bone pattern or cartilage phantom cannot be recognized.

In Fig. 14, No. 1 to No. 5 show the results of the evaluation for the images obtained by the close-contact radiographing and the phase contrast radiographing (PCM) using an Mo tube (molybdenum tube) by using the apparatus of the comparative example, and the phase contrast radiographing (PCR) using a W tube (tungsten tube). No. 6 to No. 9 show the results of the evaluation for the images obtained by the phase contrast radiographing using the apparatus in an example of the present invention.

As shown in Fig. 14, in Nos. 1 and 2 performing the close-contact radiographing using the apparatus of the comparative example, only the images on the level of finding the existence of the cartilage phantom were obtained and the bone phantom could not be recognized.

When the phase contrast radiographing (PCM) using the apparatus of the comparative example and the Mo tube (mobybdenum tube) was executed, the peripheral shape and pattern shape could be recognized clearly for the cartilage phantom, however, similarly to the close-contact radiographing, the peripheral shape and pattern shape could be recognized for the bone phantom (refer to Nos. 3 and 4).

Further, when the phase contrast radiographing (PCR) using the apparatus of the comparative example and the W tube (tungsten tube) was executed under the condition that the average radiation energy on the high energy side was set at 52 keV and the average radiation energy on the low energy side was set at 34.9 keV, the peripheral shape and pattern shape could be recognized clearly for the bone phantom, however the peripheral shape and pattern shape could not be recognized for the cartilage phantom (refer to No. 5).

On the other hand, in the embodiment of the present invention, when an experiment is conducted under the condition that the average radiation energy on the high energy side was set at 41.2 keV or higher (41.2 keV in Nos. 8 and 9, and 52 keV in Nos. 6 and 7) and the average radiation energy on the low energy side was set at 27.4 keV or lower (27.4 keV in Nos. 6 and 9, and 17.06 keV in Nos. 7 and 8), it is found that for both cartilage phantom and bone phantom, the shape and pattern can be recognized sufficiently and images acceptable for diagnosis can be obtained.

According to an embodiment of the present invention, by use of the radiation image photographing device used for the general group medical examination (periodic medical checkup), existence of deformation of the soft tissue of the cartilage part can be judged. Therefore, there is no need to use a specific apparatus such as an MRI and the effects can be produced such that an excessive burden such as expenses is not imposed on a subject and the initial symptom of the rheumatism can be diagnosed easily and precisely.

Further, two kinds of data of the phase contrast image producing the edge emphasis effect corresponding to the high-energy zone and low-energy zone are generated and the image data of the obtained image are outputted, so that a user such as a doctor confirms the outputted image, thereby can easily judge existence of an occurrence of destruction of the soft tissue which is an initial symptom of the rheumatism and existence of an occurrence of osteoporosis.

Further, according to another embodiment of the present invention, the image data of the phase contrast image corresponding to the low-energy zone and the image data of the phase contrast image corresponding to the high-energy zone can be switched and displayed properly, so that a user such as a doctor, when intending to observe the situation of the soft tissue of the cartilage part (for example, intending to judge existence of an occurrence of the initial symptom of the rheumatism), permits to display the image data of the phase contrast image corresponding to the low-energy zone and when intending to observe the situation of the bone part (for example, intending to judge existence of an occurrence of osteoporosis or existence of deformation of the bone part), permits to display the image data of the phase contrast image corresponding to the high-energy zone, thereby can diagnose easily both soft tissue and bone part.

Further, according to still another embodiment of the present invention, the average radiation energy when generating the image data of the phase contrast image corresponding to the low-energy zone is 27.4 keV or lower and the average radiation energy when generating the image data of the phase contrast image corresponding to the high-energy zone is 41.2 keV or higher, so that in the phase contrast image corresponding to the low-energy zone, the outline of the cartilage part can be demonstrated clearly, and in the phase contrast image corresponding to the high-energy zone, the bone part can be demonstrated clearly. Therefore, both a change in the cartilage part which is an initial symptom of the rheumatism and a change in the bone part such as osteoporosis which is seen when the state of disease progresses can be observed and diagnosed.

## Claims

1. An diagnosis information generating system comprising:

   a radiation image photographing device capable of generating image data of a phase contrast image corresponding to a high energy zone and image data of a phase contrast image corresponding to a low energy zone, the radiation image photographing device including:

   a radiation source for irradiating a radiation to a region of a subject to be radiographed; and

a radiation image detector for recording a radiation image according to a dose of a radiation irradiated from the radiation source and having entered the detector,

the diagnosis information generating system further comprising,
an output device for outputting image data generated by the radiation image photographing device.

2. The diagnosis information generating system of claim 1,
wherein an average radiation energy of the radiation source of the radiation image photographing device is 27.4 keV or less when generating image data of a phase contrast image corresponding to the low energy zone and the average radiation energy is 41.2 keV or more when generating image data of a phase contrast image corresponding to the high energy zone.

3. A diagnosis information generating method comprising:

generating image data of a phase contrast image corresponding to a low energy zone by irradiating a radiation to a region of a subject to be ragiographed, in a low energy zone image generating step;
generating image data of a phase contrast image corresponding to a high energy zone by irradiating a radiation to a region of a subject to be ragiographed, in a high energy zone image generating step; and
outputting the image data generated in the low energy zone image generating step and the high energy zone image generating step.

4. The diagnosis information generating method of claim 3,
wherein an average radiation energy of a radiation irradiated when generating image data of a phase contrast image corresponding to the low energy zone is 27.4 keV or less and the average radiation energy of a radiation irradiated when generating image data of a phase contrast image corresponding to the high energy zone is 41.2 keV or more.

5. A diagnosis information displaying method comprising:

generating image data of a phase contrast image corresponding to a low energy zone by irradiating a radiation to a region of a subject to be ragiographed, in a low energy zone image generating step;
generating image data of a phase contrast image corresponding to a high energy zone by irradiating a radiation to a region of a subject to be ragiographed, in a high energy zone image generating step; and
displaying the image data of a phase contrast image corresponding to a low energy zone, generated in the low energy zone image generating step and the image data of a phase contrast image corresponding to a high energy zone, generated in the high energy zone image generating step so as to be switchable to each other.

6. The diagnosis information displaying method of claim 5,
wherein an average radiation energy of a radiation irradiated when generating image data of a phase contrast image corresponding to the low energy zone is 27.4 keV or less and the average radiation energy of a radiation irradiated when generating image data of a phase contrast image corresponding to the high energy zone is 41.2 keV or more.

# FIG. 1

100

```
┌─────────────────┐
│      IMAGE      │
│   PROCESSING    │── 30
│    APPARATUS    │
└─────────────────┘
```

N

```
┌─────────────────┐        ┌─────────────────┐
│ RADIATION IMAGE │        │     IMAGE       │
│  PHOTOGRAPHING  │        │   DISPLAYING    │
│    APPARATUS    │        │    APPARATUS    │
└─────────────────┘        └─────────────────┘
```

1                              50

FIG. 2

EP 1 880 675 A1

POWER SOURCE
SECTION

EP 1 880 675 A1

FIG. 3

FIG. 4

FIG. 5

FIG. 6

FIG. 7

# FIG. 8

```
                              ┌──────────────┐
                              │   CONTROL    │──── 22
                              │    DEVICE    │
                              └──────┬───────┘
                                     │
                                     ▼
    ┌──────────────┐                        ┌──────────────┐
    │  RADIATION   │                        │    POWER     │
13 ─│     DOSE     │◄──────────────────────►│   SOURCE     │──── 9
    │  DETECTING   │                        │   SECTION    │
    │   SECTION    │                        └──────────────┘
    └──────────────┘

24 ─ ┌ ─ ─ ─ ─ ─ ─ ─ ┐
     ┌──────────────┐                        ┌──────────────┐
24a ─│ INPUT DEVICE │◄──────────────────────►│ DRIVE DEVICE │──── 6
     └──────────────┘                        └──────────────┘

     ┌──────────────┐                        ┌──────────────┐
     │   DISPLAY    │                        │    WEIGHT    │
24b ─│    DEVICE    │◄───────────────────────│    SENSOR    │──── 20
     └──────────────┘                        └──────────────┘
     └ ─ ─ ─ ─ ─ ─ ─ ┘

    ┌──────────────┐                         ┌──────────────┐
    │ INFORMATION  │                         │ RADIOGRAPHING│
26 ─│ SUPPLEMENTING│◄───────────────────────►│  DIRECTION   │──── 18
    │    DEVICE    │                         │DISCRIMINATING│
    └──────────────┘                         │    DEVICE    │
                                             └──────────────┘

                                             ┌──────────────┐
                                             │ POSITIONING  │
                              ◄─────────────►│    DEVICE    │──── 15
                                             └──────────────┘
                           23 ─
                                     │
                                     ▼
```

# FIG. 9

RADIATION ENERGY

SUBJECT

RADIATION DETECTOR

RADIATION
INTENSITY

# FIG. 10

# FIG. 11

30

CONTROL SECTION — 31

33 — INPUT SECTION

STORING SECTION — 32

34 — COMMUNICATION SECTION

IMAGE PROCESSING SECTION — 35

36

# FIG. 12 (a)

# FIG. 12 (b)

27
28
28
27

27
28
28
27

# FIG. 13

RADIATION IMAGE
PHOTOGRAPHING
APPARATUS

( START )

S1 — PHASE CONTRAST
RADIOGRAPHING

S2 — ATTACH
SUPPLEMENTARY
INFORMATION SUCH AS
SUBJECT INFORMATION
TO IMAGE DATA

S3 — TRANSMIT IMAGE DATA
WITH SUPPLEMENTARY
INFORMATION TO IMAGE
PROCESSING
APPARATUS

( END )

IMAGE PROCESSING
APPARATUS

( START )

S4 — RECEIVE IMAGE DATA
AND SUPPLEMENTARY
INFORMATION FROM
RADIATION IMAGE
PHOTOGRAPHING
APPARATUS

S5 — STORE IMAGE DATA
WITH SUPPLEMENTARY
INFORMATION IN
STORING SECTION

S6 — TRANSMIT DATA TO
IMAGE DISPLAYING
APPARATUS

( END )

IMAGE DISPLAYING
APPARATUS

( START )

S7 — RECEIVE DATA FROM
IMAGE PROCESSING
APPARATUS

S8 — DISPLAY TRANSMITTED
CONTENT IN DISPLAYING
SECTION

( END )

## FIG. 14

| No. | PADIOGRAPHING METHOD | ENLARGEMENT FACTOR | R1 [m] | R2 [m] | AVERAGE ENERGY LOW [keV] | AVERAGE ENERGY HIGH [keV] | CARTILAGE DETERMINATION | BONE DETERMINATION |
|---|---|---|---|---|---|---|---|---|
| 1 | CLOSE CONTACT | 1.00 | 0.65 | 0 | 14.9 | 20.3 | C | D |
| 2 | CLOSE CONTACT | 1.00 | 0.65 | 0 | 16.9 | 20.3 | C | D |
| 3 | PCM | 1.75 | 0.65 | 0.49 | 14.9 | 20.3 | A | D |
| 4 | PCM | 1.75 | 0.65 | 0.49 | 16.9 | 20.3 | A | D |
| 5 | PCR | 1.75 | 0.65 | 0.49 | 34.9 | 52 | D | A |
| 6 | PCR | 1.75 | 0.65 | 0.49 | 27.4 | 52 | B | A |
| 7 | PCR | 1.75 | 0.65 | 0.49 | 17.06 | 52 | A | A |
| 8 | PCR | 1.75 | 0.65 | 0.49 | 17.06 | 41.2 | A | B |
| 9 | PCR | 1.75 | 0.65 | 0.49 | 27.4 | 41.2 | B | B |

**European Patent Office**

## EUROPEAN SEARCH REPORT

Application Number

EP 07 11 1690

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 086 651 A (KONISHIROKU PHOTO IND [JP]) 28 March 2001 (2001-03-28) * paragraphs [0027], [0043]; claims 1,5; figures 1-5 * ----- | 1-6 | INV. A61B6/06 |
| A | US 2001/038707 A1 (OHARA HIROMU [JP]) 8 November 2001 (2001-11-08) * paragraph [0185]; figure 1 * ----- | 1-6 | |

TECHNICAL FIELDS SEARCHED (IPC)

A61B

The present search report has been drawn up for all claims

| Place of search | Date of completion of the search | Examiner |
|---|---|---|
| Berlin | 4 December 2007 | Bernas, Yves |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

...........................................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 07 11 1690

This annex lists the patent family members relating to the patent documents cited in the above-mentioned European search report.
The members are as contained in the European Patent Office EDP file on
The European Patent Office is in no way liable for these particulars which are merely given for the purpose of information.

04-12-2007

| Patent document cited in search report | | Publication date | Patent family member(s) | | Publication date |
|---|---|---|---|---|---|
| EP 1086651 | A | 28-03-2001 | DE<br>DE<br>US | 60021972 D1<br>60021972 T2<br>6404848 B1 | 22-09-2005<br>22-06-2006<br>11-06-2002 |
| US 2001038707 | A1 | 08-11-2001 | EP<br>JP<br>US | 1164547 A2<br>2001299733 A<br>2005213801 A1 | 19-12-2001<br>30-10-2001<br>29-09-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006192830 A
- JP 2001091479 A
- JP 2002162705 A
- JP 2004248699 A
- JP 5211635 A